(19) Europäisches Patentamt
European Patent Office
Office européen des brevets

(11) Veröffentlichungsnummer: **0 113 318**
**B1**

(12) # EUROPÄISCHE PATENTSCHRIFT

(45) Veröffentlichungstag der Patentschrift: **16.09.87**

(51) Int. Cl.⁴: **C 07 D 207/416**, C 08 K 5/37

(21) Anmeldenummer: **83810619.3**

(22) Anmeldetag: **23.12.83**

(54) **Neue (4-Hydroxyphenylthio)-imid Stabilisatoren.**

(30) Priorität: **29.12.82 US 454214**

(43) Veröffentlichungstag der Anmeldung: **11.07.84 Patentblatt 84/28**

(45) Bekanntmachung des Hinweises auf die Patenterteilung: **16.09.87 Patentblatt 87/38**

(84) Benannte Vertragsstaaten: **BE DE FR GB IT NL**

(56) Entgegenhaltungen:
**DE - A - 1 445 084**
**FR - A - 2 132 852**
**US - A - 3 625 978**
**US - A - 3 832 329**

(73) Patentinhaber: **CIBA-GEIGY AG, Klybeckstrasse 141, CH-4002 Basel (CH)**

(72) Erfinder: **Spivack, John D., 1 Blue Jay Street, Spring Valley New York 10977 (US)**
Erfinder: **Pastor, Stephen D., 112 Union Road, Spring Valley New York 10977 (US)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 113 318 B1

ACTORUM AG

**Beschreibung**

Die Erfindung betrifft neue Mercaptophenolderivate, deren Verwendung als Stabilisatoren für organisches Material sowie die damit stabilisierten Zusammensetzungen.

Organische polymere Verbindungen wie Kunststoffe oder Harze unterliegen thermischem, oxidativem oder lichtinduziertem Abbau. In der Technik ist eine grosse Anzahl von Stabilisatoren für eine Vielzahl von Substraten bekannt. Die Wirksamkeit eines Stabilisators hängt unter anderem von der Art des Substrats, in welchem er eingesetzt wird, und vom jeweiligen Abbaumechanismus ab. Daher ist es im allgemeinen schwierig, für eine konkrete Anwendung den wirksamsten und ökonomischsten Stabilisator anzugeben.

So kann beispielsweise ein Stabilisator, der die Flüchtigkeit einer Verbindung reduziert, dahingehend wirken, dass er einen Bindungsbruch der Substratmoleküle verhindert. Um eine geringe Versprödung oder die Erhaltung der Elastizität eines Polymeren oder eines Elastomeren zu gewährleisten, kann von einem Stabilisator verlangt werden, dass er übermässige Vernetzungsreaktionen und/oder Kettenbruch verhindert. Um die Vergilbung eines Substrats zu unterbinden, muss verhindert werden, dass Reaktionen des Substrats oder des Stabilisators ablaufen, die zu neuen Chromophoren führen. Weiterhin müssen Probleme der Verarbeitungsstabilität und der Substratverträglichkeit beachtet werden.

Aus FR-A 2 132 852 sind 1-(p-Hydroxyphenyl)-3-benzylthiomaleinimide als Stabilisatoren gegen thermischen und oxidativen Abbau in organischen Materialien bekannt. In der US-PS 3 625 978 werden Hydroxyarylthioimide beschrieben, die sich als Comonomere für Polykondensationsreaktionen eignen.

Überraschenderweise ist jetzt gefunden worden, dass die (Hydroxyphenylthio)-imid Derivate dieser Erfindung eine ungewöhnliche Kombination wünschenswerter Eigenschaften besitzen, wodurch diese Stoffe zu besonders effektiven und nützlichen Stabilisatoren werden. Die erfindungsgemässen Verbindungen erweisen sich als besonders nützliche Stabilisatoren für schlagfestes Polystyrol, Elastomere wie Polybutadien oder Styrol-Butadien-Elastomere, bei denen der Erhalt der Elastizität und die Verhinderung von Vernetzungsreaktionen, von Verfärbung, von Geruchsbildung und von Ausschwitzen des Stabilisators grundlegende Anforderungen an die Qualität eines Stabilisators sind.

Die Erfindung betrifft Verbindungen der Formel I

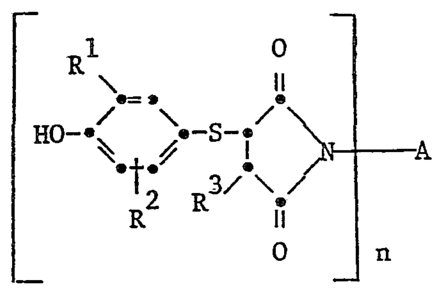 (I),

worin $R^1$ und $R^2$ unabhängig voneinander Wasserstoff, $C_1$–$C_{18}$Alkyl, $C_5$–$C_6$Cycloalkyl, Phenyl oder mit $C_1$–$C_{12}$Alkyl substituiertes Phenyl bedeuten, worin $R^3$ Wasserstoff oder $C_1$–$C_3$Alkyl bedeutet, n 1, 2 oder 3 ist, und worin a, je nach Wert von n, folgende Bedeutung zukommt:

a) wenn n 1 ist, bedeutet A Phenyl, mit ein oder zwei $C_1$–$C_{18}$Alkylgruppen substituiertes Phenyl, $C_1$–$C_{30}$Alkyl, $C_5$ oder $C_6$ Cycloalkyl, oder A ist ein Rest vom Typ E-(G-T)$_y$-, worin E $C_1$–$C_{18}$Alkyl bedeutet, G -O- oder -NH- ist, T Ethylen, Propylen oder 1,4-Butylen bedeutet und y eine Zahl von 1 bis 30 ist;

b) wenn n 2 ist, bedeutet A Phenylen, durch ein oder zwei $C_1$–$C_{12}$Alkylgruppen substituiertes Phenylen, $C_1$–$C_{12}$Alkylen, $C_5$ oder $C_6$ Cycloalkylen, Phenylen-L-Phenylen, worin L $C_1$–$C_4$Alkylen bedeutet, oder A ist -T-(G-T)$_z$-, worin T Ethylen, Propylen oder 1,4-Butylen bedeutet, G -O- oder -NH- ist, und z eine Zahl von 1 bis 30 ist;

c) wenn n 3 ist, bedeutet A Benzol-1,2,3-, -1,2,4- oder -1,3,5-triyl oder N(-$R^4$-)$_3$, worin $R^4$ $C_2$–$C_4$Alkylen bedeutet.

Bedeutet einer der Reste gegebenenfalls $C_1$–$C_3$Alkyl, so handelt es sich beispielsweise um Methyl, Ethyl, n-Propyl oder Isopropyl. Als $C_1$–$C_{18}$Alkyl können diese Reste zusätzlich noch n-Butyl, sek.-Butyl, tert.-Butyl, n-Pentyl, Isoamyl, n-Hexyl, 1,1,3,3-Tetramethylbutyl, n-Octyl, n-Decyl, 1,1,3,3,5,5-Hexamethylhexyl, n-Dodecyl, n-Tetradecyl, n-Hexadexyl oder n-Octadecyl bedeuten. Als $C_1$–$C_{30}$Alkyl können diese Reste gegebenenfalls noch Eicosanyl, Docosanyl oder Tricontyl sein.

Bedeutet einer der Reste gegebenenfalls $C_5$–$C_6$Cycloalkyl, so handelt es sich um Cyclopentyl oder um Cyclohexyl.

Als mit $C_1$–$C_{12}$Alkyl substituiertes Phenyl bedeuten die Reste $R_1$, $R_2$ oder A beispielsweise o-, m- oder p-Tolyl, Xylyl, Ethylphenyl, n-Propylphenyl, n-Butylphenyl, n-Pentylphenyl, n-Hexylphenyl, n-Octylphenyl, n-Decylphenyl, n-Dodecylphenyl, Isopropylphenyl, sek.-Butylphenyl, tert.-Butylphenyl, 1,1,3,3-Tetramethylbutylphenyl, sowie Diethylphenyl, Di-n-propylphenyl, Di-n-butylphenyl, Di-n-pentylphenyl, Di-n-hexylphenyl, Di-n-octylphenyl, Di-n-decylphenyl, Di-n-dodecylphenyl oder Di-(1,1,3,3-tetramethylbutyl)-phenyl. Dabei befinden sich bei monoalkylsubstituierten Phenylgruppen die Substituenten in ortho-, meta- oder para-Position, während sie sich bei dialkylsubstituierten Phenylgruppen in 2,4-, 2,5- oder 2,6-Position befinden.

Als $C_2$–$C_4$Alkylen bedeutet $R^4$ beispielsweise Ethylen, Propan-1,1-, -1,2- oder -1,3-diyl. Als $C_1$–$C_{12}$Alkylen bedeutet A zusätzlich Methylen, Butan-1,1-, -1,2-, -1,3- oder -1,4-diyl, Pentan-1,5-diyl, Hexan-1,6-diyl, Octan-1,8-diyl, Decan-1,10-diyl oder Dodecan-1,12-diyl. Als $C_5$–$C_6$Cycloalkylen bedeutet A beispielsweise Cyclopentan-1,2- oder -1,3-diyl oder es ist Cyclohexan-1,2-, -1,3- oder -1,4-diyl.

Als mit ein oder zwei $C_1$–$C_{12}$Alkylgruppen substituiertes Phenylen bedeutet A beispielsweise 1,4-(2-Methyl)-phenylen, 1,3-(2-Methyl)-phenylen, 1,3-(4-Methyl)-phenylen, 1,3-(5-Methyl)-phenylen, 1,3-(6-Methyl)-phenylen, 1,2-(3-Methyl)-phenylen, 1,2-(4-Methyl)-phenylen, 1,2-(5-Methyl)-phenylen, 1,2-(6-Methyl)-phenylen, 1,4-(2-Ethyl)-phenylen, 1,4-(2-n-Propyl)-phenylen, 1,4-(2-Isopropyl)-phenylen, 1,4-(2-n-Butyl)-phenylen, 1,4-(2-sek.Butyl)-phenylen, 1,4-(2-tert.Butyl)-phenylen, 1,4-(2-n-Hexyl)-phenylen, 1,4-(2-n-Octyl)-phenylen, 1,4-(2-n-Decyl)-phenylen oder 1,4-(2-n-Dodecyl)-phenylen, sowie 1,4-(2,3-Dimethyl)-phenylen, 1,4-(2,5-Dimethyl)-phenylen, 1,4-(2,6-Dimethyl)-phenylen, 1,4-(2,5-Diethyl)-phenylen, 1,4-(2,5-Di-n-propyl)-phenylen, 1,4-(2,5-Di-n-butyl)-phenylen, 1,4-(2,5-Di-sek.butyl)-phenylen, 1,4-(2,5-Di-tert.butyl)-phenylen, 1,4-(2,5-Di-n-hexyl)-phenylen, 1,4-(2,5-Di-n-octyl)-phenylen, 1,4-(2,5-Di-n-decyl)-phenylen oder 1,4-(2,5-Di-n-dodecyl)-phenylen.

Die Gruppen $R^1$ und/oder $R^2$ sind vorzugsweise geradkettiges oder verzweigtes $C_4$–$C_8$Alkyl, wie beispielsweise n-Butyl, sek.Butyl, tert.Butyl, tert.Pentyl, 2-Ethylhexyl, n-Octyl oder 1,1,3,3-Tetramethylbutyl. Besonders bevorzugt werden $R^1$ und/oder $R^2$ als tert.Butyl, tert.Pentyl oder 1,1,3,3-Tetramethylbutyl.

Ebenfalls bevorzugt werden Verbindungen der Formel I, bei denen die Gruppe $R^2$ sich in o-Position zur Hydroxygruppe befindet, insbesondere wenn $R^2$ tert.Alkyl ist, und besonders bevorzugt wenn $R^2$ $C_4$–$C_8$ tert.Alkyl bedeutet. Wenn $R^1$ und/oder $R^2$ substituiertes Phenyl bedeuten, so behandelt es sich bei den Substituenten vorzugsweise um $C_1$–$C_8$Alkyl.

Im Falle von n = 1 bedeutet A vorzugsweise Phenyl oder $C_1$–$C_{12}$Alkyl, während im Fall von n = 2 A vorzugsweise Hexamethylen, Phenylen oder 4,4-Methylendiphenylen bedeutet, und schliesslich ist A im Fall n = 3 bevorzugt 1,3,5-trisubstituiertes Benzol oder $N(CH_2CH_2-)_3$.

Die Imide der vorliegenden Erfindung stellt man durch Reaktion der entsprechend substituierten Maleinimide mit einem alkylierten Mercaptophenol her. Die Reaktionsdurchführung erfolgt gegebenenfalls in einem geeigneten Lösungsmittel, das ein aromatischer Kohlenwasserstoff wie Benzol, Toluol, Xylol oder ein heterozyklischer Ether wie Tetrahydrofuran sein kann. Die Reaktionstemperatur liegt zwischen Raumtemperatur und 70°C. In einer bevorzugten Ausführungsform lässt man das Maleinimid mit dem Mercaptophenol in Gegenwart eines Protonenakzeptors wie beispielsweise einem tertiären Amin, bevorzugt Triethylamin, oder Pyridin reagieren.

Die Ausgangsverbindungen zur Herstellung der Imide der Formel I sind im Handel erhältlich oder können nach bekannten Verfahren hergestellt werden. So werden beispielsweise die substituierten Maleinimide durch Reaktion von Ammoniak oder primären Aminen mit einem zyklischen Anhydrid wie beispielsweise Maleinsäure- oder Citraconsäureanhydrid erhalten. Einen Überblick über die präparativen Methoden zur Herstellung zyklischer Imide geben Hargreaves, Pritchard und Dave in Chemical Reviews, Vol. 70, 439–469 (1970). Typische Maleinimide, worin $R^3$ Wasserstoff bedeutet, sind beispielsweise N,N-Hexamethylen-bis-(maleinimid), N-Phenylmaleinimid, N-Methylmaleinimid, 1,4-Bis-(maleinimido)-benzol, N-n-Butylmaleinimid, N,N-o-Phenylendimaleinimid, N-Dodecylmaleinimid, 4,4-Bis-(maleinimido)-diphenylmethan und N-Cyclohexylmaleinimid. Ähnlich strukturierte Citraconimide, worin $R^3$ Methyl bedeutet, sind ebenfalls wichtige Ausgangsstoffe zur Synthese der erfindungsgemässen Verbindungen. Als Mercaptophenole verwendet man Verbindungen der Formel II

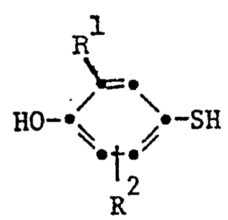

(II),

worin $R^1$ und $R^2$ die oben definierte Bedeutung besitzen.

Bevorzugte 4-Mercaptophenole sind beispielsweise 2,6-Di-tert.butyl-, 2-tert.Butyl-6-methyl-, 2,6-Dimethyl- oder 2-tert.Butyl-5-methyl-4-mercaptophenol.

Die erfindungsgemässen Verbindungen lassen sich als effektive Stabilisatoren für Kunststoffe, Polymere oder Harze einsetzen.

Die Erfindung betrifft daher auch eine Zusammensetzung enthaltend ein gegen oxidativen, thermischen oder strahlungsinduzierten Abbau empfindliches organisches Material und mindestens eine Verbindung der Formel I als Stabilisator, wobei als organisches Material vorzugsweise ein Polymer zu verstehen ist, besonders bevorzugt aber ein polyolefinisches Homo- oder Copolymer, insbesondere aber auch ein Homopolymer, Copolymer oder Terpolymer von Styrol.

Vorzugsweise verwendet man die Verbindungen der Formel I als Stabilisatoren für Polyolefine wie beispielsweise Polyethylen, Polypropylen, Polyisobutylen, Poly-(buten-1), Poly-(penten-1), Poly-(3-methylbuten-1), Poly-(4-methylpenten-1), unterschiedliche Ethylen-Propylen-Copolymere, Polystyrol, schlagfestes Polystyrol, ABS, SBR, Polyisopren, natürlichen Kautschuk, Polyester wie Polyethylenterephthalat oder Polybutylenterephthalat sowie Copolymere davon. Die Verbindungen der Formel I eignen sich auch als Stabilisatoren für Polyurethane, Polycarbonate, Polyamide, wie beispielsweise Nylon 6, 6/6 und andere Typen, sowie für Copolyamide oder Polysulfone.

Polymere, die mit den erfindungsgemässen Verbindungen stabilisiert werden können, sind beispielsweise:

1. Polymere von Mono- und Diolefinen, beispielsweise Polyethylen (das gegebenenfalls vernetzt sein kann), Polypropylen, Polyisobutylen, Polybuten-1, Polymethylpenten-1, Polyisopren oder Polybutadien sowie Polymerisate von Cycloolefinen wie z.B. von Cyclopenten oder Norbornen.

2. Mischungen der unter 1) genannten Polymeren, z.B. Mischungen von Polypropylen mit Polyisobutylen.

3. Copolymere von Mono-und Diolefinen untereinander oder mit anderen Vinylmonomeren, wie z.B. Ethylen-Propylen-Copolymere, Propylen-Buten-1-Copolymere, Propylen-Isobutylen-Copolymere, Ethylen-Buten-1-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen.

4. Polystyrol, Poly-(p-methylstyrol).

5. Copolymere von Styrol oder α-Methylstyrol mit Dienen oder Acrylderivaten, wie z.B. Styrol-Butadien, Styrol-Acrylnitril, Styrol-Alkylmethacrylat, Styrol-Maleinanhydrid, Styrol-Acrylnitril-Methylacrylat; Mischungen von hoher Schlagzähigkeit aus Styrol-Copolymeren und einem anderen Polymer wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

6. Pfropfcopolymere von Styrol, wie z.B. Styrol auf Polybutadien, Styrol und Acrylnitril auf Polybutadien, Styrol und Maleinsäureanhydrid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z.B. als sogennante ABS, MBS, ASA oder AES-Polymere bekannt sind.

7. Halogenhaltige Polymere, wie z.B. Polychloropren, Chlorkautschuk, chloriertes oder chlorsulfoniertes Polyethylen, Epichlorhydrinhomo- und-copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere wie Vinylchlorid-Vinylidenchlorid, Vinylchlorid-Vinylacetat oder Vinylidenchlorid-Vinylacetat.

8. Polymere, die sich von α,β-ungesättigten Säuren und deren Derivaten ableiten, wie Polyacrylate und Polymethacrylate, Polyacrylamide und Polyacrylnitrile.

9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat-, -stearat, -benzoat, -maleat, Polyvinylbutyral, Polyallylphthalat, Polyallylmelamin.

11. Homo- und Copolymere von cyclischen Ethern, wie Polyalkylenglykole, Polyethylenoxyd, Polypropylenoxyd oder deren Copolymere mit Bisglycidylethern.

12. Polyacetale, wie Polyoxymethylen, sowie solche Polyoxymethylene, die Comonomere wie z.B. Ethylenoxid enthalten.

13. Polyphenyloxide und -sulfide und deren Mischungen mit Styrolpolymeren.

14. Polyurethane, die sich von Polyethern, Polyestern und Polybutadienen mit endständigen Hydroxylgruppen einerseits und aliphatischen oder aromatischen Polyisocyanaten andererseits ableiten sowie deren Vorprodukte.

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, Polyamid 6/10, Polyamid 11, Polyamid 12, Poly-2,4,4-trimethylhexamethylenterephthalamid, Poly-m-phenylen-isophthalamid, sowie deren Block-Copolymere mit Polyethern wie z.B. mit Polyethylenglykol, Polypropylenglykol oder Polytetramethylenglykol.

16. Polyharnstoffe, Polyimide, Polyamid-imide und Polybenzimidazole.

17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyetherester, die sich von Polyethern mit Hydroxylendgruppen ableiten.

18. Polycarbonate.

19. Polysulfone, Polyethersulfone und Polyetherketone.

20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

23. Vernetzbare Acrylharze, die sich von substituierten Acrylsäureestern ableiten wie z.B. von Epoxyacrylaten, Urethan-acrylaten oder Polyester-acrylaten.

24. Alkydharze, Polyesterharze und Acrylatharze, die mit Melaminharzen, Harnstoffharzen, Polyisocyanaten, oder Epoxidharzen vernetzt sind.

25. Vernetzte Epoxidharze, die sich von Polyepoxiden ableiten, z.B. von Bis-glycidylethern, von cycloaliphatischen Diepoxiden oder von aromatischen Diepoxiden.

26. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose.

27. Mischungen (Polyblends) der vorgenannten Polymeren wie z.B. PP/EPDM, Polyamid 6/EPDM oder ABS, PVC/EVA, PVC/ABS, PVC/MBS, PC/ABS, PBTP/ABS.

28. Natürliche und synthetische organische Stoffe, die reine monomere Verbindungen oder Mischungen von solchen darstellen, beispielsweise Mineralöle, tierische oder pflanzliche Fette, Öle und Wachse, oder Öle, Wachse und Fette auf Basis synthetischer Ester (z.B. Phthalate, Adipate, Phosphate oder Trimellithate), sowie Abmischungen synthetischer Ester mit Mineralölen in beliebigen Gewichtsverhältnissen, wie sie z.B. als Spinnpräparationen Anwendung finden, sowie deren wässrigen Emulsionen.

29. Wässrige Emulsionen natürlicher oder synthetischer Kautschuke, wie z.B. Naturkautschuk-Latex oder Latices von carboxylierten Styrol-Butadien-Copolymeren.

Die Erfindung betrifft weiterhin ein Verfahren zum Stabilisieren von organischem Material gegen oxidativen, thermischen oder strahlungsinduzierten Abbau, dadurch gekennzeichnet, dass man dem Material mindestens eine Verbindung der Formel I wie oben beschrieben zusetzt.

Die erfindungsgemässen Stabilisatoren werden den Kunststoffen in Mengen von 0,01 bis 5 Gew.% bezogen auf das stabilisierte Material zugemischt. Je nach Verwendung und Substrat wird die Stabilisatormenge variiert. Bevorzugt verwendet man 0,05 bis 2 Gew.% des Stabilisators, besondes bevorzugt 0,1 bis 1 Gew.%.

Die Einarbeitung der Stabilisatorsubstanzen in die organischen Polymeren erfolgt nach bekannten Verfahren. Die Zudosierung kann während jeder Verarbeitungsstufe vor der Formgebung erfolgen. So kann der Stabilisator mit den pulverförmigen Polymeren gemischt werden, oder eine Emulsion oder eine Suspension des Stabilisators kann mit einer Lösung, Suspension oder Emulsion des Polymeren vermischt werden.

Die so stabilisierten Materialien können in verschiedenster Form angewendet werden, z.B. als Folien, Fasern, Bändchen, Formmassen, Profile oder als Bindemittel für Lacke, Klebstoffe oder Kitte.

Die stabilisierten Polymerzusammensetzungen können gegebenenfalls noch weitere Zusätze enthalten, wie zum Beispiel:

1. Antioxidantien

1.1. Alkylierte Monophenole
2,6-Di-tert.butyl-4-methylphenol
2-Tert.butyl-4,6-dimethylphenol
2,6-Di-tert.butyl-4-ethylphenol
2,6-Di-tert.butyl-4-n-butylphenol
2,6-Di-tert.butyl-4-i-butylphenol
2,6-Di-cyclopentyl-4-methylphenol
2-(α-Methylcyclohexyl)-4,6-dimethylphenol
2,6-Di-octadecyl-4-methylphenol
2,4,6-Tri-cyclohexylphenol
2,6-Di-tert.butyl-4-methoxymethylphenol.

1.2. Alkylierte Hydrochinone
2,6-Di-tert.butyl-4-methoxyphenol
2,5-Di-tert.butyl-hydrochinon
2,5-Di-tert.amyl-hydrochinon
2,6-Diphenyl-4-octadecyloxyphenol
2,5-di-tert.butyl-hydrochinon
3,5-Di-tert.butyl-4-hydroxyanisol
Tris-(3,5-di-tert.butyl-4-hydroxyphenyl)-phosphit
3,5-Di-tert.butyl-4-hydroxyphenylstearat
Bis-(3,5-di-tert.butyl-4-hydroyphenyl)-adipat.

1.3. Hydroxylierte Thiodiphenylether
2,2,'-Thio-bis-(6-tert.butyl-4-methylphenol)
2,2'-Thio-bis-(4-octylphenol)
4,4'-Thio-bis-(6-tert.butyl-3-methylphenol)
4,4'-Thio-bis-(6-tert.butyl-2-methylphenol)
4,4'-Thio-bis-(3,6-di-sek.amylphenol)
4,4'-Bis-(2,6-dimethyl-4-hydroxyphenyl)-disulfid.

1.4. Alkyliden-Bisphenole
2,2'-Methylen-bis-(6-tert.butyl-4-methylphenol)
2,2'-Methylen-bis-(6-tert.butyl-4-ethylphenol)
2,2'-Methylen-bis-[4-methyl-6-(α-methylcyclohexyl)-phenol]
2,2'-Methylen-bis-(4-methyl-6-cyclohexylphenol)
2,2'-Methylen-bis-(6-nonyl-4-methylphenol)
2,2'-Methylen-bis-(4,6-di-tert.butylphenol)
2,2'-Ethyliden-bis-(4,6-di-tert.butylphenol)
2,2'-Ethyliden-bis-(6-tert.butyl-4-isobutylphenol)
2,2'-Methylen-bis-[6-(α-methylbenzyl)-4-nonylphenol]
2,2'-Methylen-bis-[6-(α,α-dimethylbenzyl)-4-nonylphenol]
4,4'-Methylen-bis-(2,6-di-tert.butylphenol)
4,4'-Methylen-bis-(6-tert.butyl-2-methylphenol)
1,1-Bis-(5-tert.butyl-4-hydroxy-2-methylphenyl)-butan
2,6-Di-(3-tert.butyl-5-methyl-2-hydroxybenzyl)-4-methylphenol

1,1,3-Tris-(5-tert.butyl-4-hydroxy-2-methylphenyl)-butan
1,1-Bis-(5-tert.butyl-4-hydroxy-2-methylphenyl)-3-n-dodecylmercaptobutan
Ethylenglycol-bis-[3,3-bis-(3′-tert.butyl-4′-hydroxyphenyl)-butyrat]
Di-(3-tert.butyl-4-hydroxy-5-methylphenyl)-dicyclopentadien
Di-[2-(3′-tert.butyl-2′-hydroxy-5′-methyl-benzyl)-6-tert.butyl-4-methyl-phenyl]-terephthalat
1,1-Bis-(3,5-dimethyl-2-hydroxyphenyl)-butan
2,2-Bis-(3,5-Di-tert.butyl-4-hydroxyphenyl)-propan
1,1,5,5-Tetra-(5-tert.butyl-4-hydroxy-2-methylphenyl)-pentan.

1.5. O-, N- und S-Benzyl Verbindungen
3,3,5,5-Tetra-tert.butyl-4,4-dihydroxydibenzylether
Octadecyl-4-hydroxy-3,5-dimethylbenzylmercaptoacetat
Tris-(3,5-di-tert.butyl-4-hydroxybenzyl)-amin
Bis-(4-tert.butyl-3-hydroxy-2,6-dimethylbenzyl)-dithioterephthalat.

1.6. Hydroxybenzylierte Malonate
Di-octadecyl-2,2-bis-(3,5-di-tert.butyl-2-hydroxybenzyl)-malonat
Di-octadecyl-2-(3-tert.butyl-4-hydroxy-5-methylbenzyl)-malonat
Di-dodecylmercaptoethyl-2,2-bis-(3,5-di-tert.butyl-4-hydroxybenzyl)-malonat
Di-[4-(1,1,3,3-tetramethylbutyl)-phenyl]-2,2-bis-(3,5-di-tert.butyl-4-hydroxybenzyl)-malonat.

1.7. Benzylverbindungen
1,3,5-Tri-(3,5-di-tert.butyl-4-hydroxybenzyl)-2,4,6-trimethylbenzol
Di-(3,5-di-tert.butyl-4-hydroxybenzyl)-sulfid
3,5-Di-tert.butyl-4-hydroxybenzyl-mercaptoessigsäure-isooctylester
Bis-(4-tert.butyl-3-hydroxy-2,6-dimethylbenzyl)dithiol-terephthalat
1,3,5-Tris-(3,5-di-tert.butyl-4-hydroxybenzyl)-isocyanurat
1,3,5-Tris-(4-tert.butyl-3-hyxdroxy-2,6-dimethylbenzyl)-isocyanurat
3,5-Di-tert.butyl-4-hydroxybenzyl-phosphonsäure-dioctadecylester
3,5-Di-tert.butyl-4-hydroxybenzyl-phosphonsäure-monoethylester, Calcium-salz
1,4-Bis-(3,5-di-tert.butyl-4-hydroxybenzyl)-2,3,5,6-tetramethylbenzol
2,4,6-Tris-(3,5-di-tert.butyl-4-hydroxybenzyl)-phenol.

1.8. s-Triazinverbindungen
2,4-Bis-octylmercapto-6-(3,5-di-tert.butyl-4-hydroxyanilino)-s-triazin
2-Octylmercapto-4,6-bis-(3,5-di-tert.butyl-4-hydroxyanilino)-s-triazin
2-Octylmercapto-4,6-bis-(3,5-di-tert.butyl-4-hydroxyphenoxy)-s-triazin
2,4,6-Tris-(3,5-di-tert.butyl-4-hydroxyphenylethyl)-s-triazin
1,3,5-Tris-(3,5-di-butyl-4-hydroxyphenylethyl)-s-triazin
1,3,5-Tris-(3,5-di-tert.butyl-4-hydroxybenzyl)-isocyanurat.

1.9. Acylaminophenole
4-Hydroxy-laurinsäureanilid
4-Hydroxy-stearinsäureanilid
2,4-Bis-octylmercapto-6-(3,5-di-tert.butyl-4-hydroxyanilino)-s-triazin
N-(3,5-di-tert.butyl-4-hydroxyphenyl)-carbaminsäureoctylester.

1.10. Ester der β-(3,5-Di-tert.butyl-4-hydroxyphenyl)-propionsäure mit ein- oder mehrwertigen Alkoholen, wie z.B. mit

| | |
|---|---|
| Methanol | Diethylenglycol |
| Octadecanol | Triethylenglycol |
| 1,6-Hexandiol | Pentaerythrit |
| Neopentylglycol | Tris-hydroxyethyl-isocyanurat |
| Thiodiethylenglycol | |
| Ethanol | Di-hydroxyethyl-oxalsäurediamid |
| Ethylenglykol | |
| 3-Thia-undecanol | 1,9-Nonadiol |
| Trimethylhexandiol | 1,2-Propandiol |
| Trimethylolpropan | 3-Thia-pentadecanol |
| | Trimethylolethan |

4-Hydroxymethyl-1-phospha-2,6,7-trioxabicyclo-[2,2,2]-octan

1.11. Ester der β(5-tert.butyl-4-hydroxy-3-methylphenyl)-propionsäure mit ein- oder mehrwertigen Alkoholen, wie z.B. mit

| | |
|---|---|
| Methanol | Diethylenglycol |
| Octadecanol | Triethylenglycol |
| 1,6-Hexandiol | Pentaerythrit |
| Neopentylglycol | Tris-hydroxyethyl-isocyanurat |
| Thiodiethylenglycol | |
| Ethanol | Di-hydroxyethyl-oxalsäurediamid |
| Ethylenglykol | |
| 3-Thia-undecanol | 1,9-Nonandiol |
| Trimethylhexandiol | 1,2-Propandiol |
| Trimethylolpropan | 3-Thia-pentadecanol |
| | Trimethylolethan |

4-Hydroxymethyl-1-phospha-2,6,7-trioxabicyclo-[2,2,2]-octan

1.12. Amide der β-(3,5-Di-tert.butyl-4-hydroxyphenyl)-propionsäure, wie z.B.
N,N′-Di-(3,5-di-tert.butyl-4-hydroxyphenylpropionyl)-hexamethylendiamin
N,N′-Di-(3,5-di-tert.butyl-4-hydroxyphenylpropionyl)-trimethylendiamin
N,N′-Di-(3,5-di-tert.butyl-4-hydroxyphenylpropionyl)-hydrazin
N,N′-Di-(3,5-di-tert.butyl-4-hydroxyphenylpropionyl)-hexamethylendiamin
N,N-Bis-β-(3,5-di-tert.butyl-4-hydroxyphenylpropionyl)-isocyanurat

1.13. Ester der 3,5-Di-tert.butyl-4-hydroxyphenylessigsäure mit ein- oder mehrwertigen Alkoholen wie z.B.:

| | |
|---|---|
| Methanol | Ethanol |
| Octadecanol | 1,6-Hexandiol |
| 1,9-Nonandiol | Ethylenglycol |
| 1,2-Propandiol | Diethylenglycol |
| Thiodiglycol | Neopentylglycol |
| Pentaerithrit | 3-Thia-undecanol |
| 3-Thia-pentadecanol | Trimethylhexandiol |
| Trimethylolethan | Trimethylolpropan |
| Tris-hydroxyethyl-isocyanurat | |

4-Hydroxymethyl-1-phospha-2,6,7-trioxabicyclo-[2,2,2]-octan und besonders bevorzugt der Tetra-bis Ester mit Pentaerithrit.

1.14. Benzylphosphonate wie zum Beispiel:
Dimethyl-3,5-di-tert.butyl-4-hydroxybenzyl-phosphonat
Diethyl-3,5-di-tert.butyl-4-hydroxybenzyl-phosphonat
Dioctadecyl-3,5-di-tert.butyl-4-hydroxybenzyl-phosphonat
Dioctadecyl-5-tert.butyl-4-hydroxy-3-methyl-benzylphosphonat.

Im folgenden sind Beispiele von weiteren Additiven aufgezählt, die zusammen mit den oben erwähnten Antioxidantien und den erfindungsgemässen Stabilisatoren eingesetzt werden können. Zu diesen Stoffen zählen beispielsweise:

2. UV-Absorber und Lichtschutzmittel
2.1. 2-(2'-Hydroxyphenyl)-benztriazole, wie z.B. das 5'-Methyl-3',5'-Di-tert.butyl-, 5'-Tert.butyl-, 5'-(1,1,3,3-Tetramethylbutyl)-, 5-Chlor-3',5'-di-tert.butyl-, 5-Chlor-3'-tert.butyl-5'-methyl-, 3'-sec.Butyl-5'-tert.butyl, 4'-Octoxy-, 3',5'-Di-tert.amyl-, 3',5'-Bis-(α,α-dimethylbenzyl)-, 3'--Methylbenzyl-5'-methyl-5-chlor-, 4'-Hydroxy-, 4'-Methoxy-, 3'-Methyl-5'-carbomethoxyethyl-, 3',5'-Bis-(α,α-dimethylbenzyl)-5-chlor-, 3'5'-Di-tert.octylphenyl, 3',5'-Di-tert.-octylphenyl-5-chlor- oder 5-Chlor-3',5'-di-tert.amyl-Derivate.

2.2. 2,4-Bis-(2-hydroxyphenyl)-6-alkyl-s-triazine wie beispielsweise das 6-Ethyl-, 6-Heptadecyl- oder das 6-Undecyl-Derivat.

2.3. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

2.4. 1,3-Bis-(2'-hydroxybenzoyl)-benzole wie beispielsweise:
1,3-Bis-(2'-hydroxy-4'-hexyloxybenzoyl)-benzol
1,3-Bis-(2'-hydroxy-4'-octyloxybenzoyl)-benzol
1,3-Bis-(2'-hydroxy-4'-dodecyloxybenzoyl)-benzol.

2.5. Ester von gegebenenfalls substituierten Benzoesäuren, wie z.B. 4-Tert.butyl-phenylsalicylat, Phenylsalicylat, Octylphenylsalicylat, Dibenzoylresorcin, Bis-(4-tert.butylbenzoyl)-resorcin, Benzoylresorcin, 3,5-Di-tert.butyl-4-hydroxybenzoesäure-2,4-di-tert.butylphenylester, 3,5-Di-tert.butyl-4-hydroxybenzoesäurehexadecylester oder der n-Octadecylester oder der 2-methyl-4,6-di-tert.butylester

2.6. Acrylate, wie z.B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäuremethylester, N-(β-Carbomethoxy-β-cyanovinyl)-2-methyl-indolin.

2.7. Nickelverbindungen, wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden wie n-Butylamin, Triethanolmin oder N-Cyclohexyldiethanolamin, Nickeldibutyldithiocarbamat, Nickelsalze von 4-Hydroxy-3,5-di-tert.butylbenzylphosphonsäure-monoalkylestern wie vom Methyl- oder Ethylester, Nickelkomplexe von Ketoximen wie von 2-Hydroxy-4-methyl-phenyl-undecylketonoxim, Nickelkomplexe des 1-Phenyl-4-lauroyl-5-hydroxy-pyrazols, gegebenenfalls mit zusätzlichen Liganden.

2.8. Sterisch gehinderte Amine, wie z.B. Bis-(2,2,6,6-tetramethylpiperidyl)-sebacat
Bis-(1,2,2,6,6-pentamethylpiperidyl)-sebacat
n-Butyl-3,5-di-tert.butyl-4-hydroybenzyl-malonsäure-bis(1,2,2,6,6-pentamethylpiperidyl)-ester, Kondensationsprodukt aus 1-Hydroxyethyl-2,2,6,6-Tetramethyl-4-hydroxypiperidin und Bernsteinsäure, Kondensationsprodukt aus N,N'-(2,2,6,6-Tetramethylpiperidyl)-hexamethylendiamin und 4-tert.octylamino-2,6-dichlor-1,3,5-s-triazin,
Tris-(2,2,6,6-tetramethylpiperidyl)-nitrilotriacetat,
Tetrakis-(2,2,6,6-tetramethyl-4-piperidyl)-1,2,3,4-butantetracarbonsäure,
1,1'-(1,2-ethandiyl)-bis-(3,3,5,5-tetramethyl-piperazinon)
4-Benzoyl-2,2,6,6-tetramethylpiperidin, 4-Stearyl-oxy-2,2,6,6-tetramethylpiperidin, 3-n-Octyl-7-7-9-9-tetramethyl-1,3,8-triaza-spirs[4,5]-decan-2,4-dion.

2.9. Oxalsäurediamide, wie z.B. 4,4'-Di-octyloxyoxanilid, 2,2'-Di-octyloxy-5,5'-di-tert.butyl-oxanilid, 2,2'-Di-dodecyloxy-5,5'-di-tert.butyl-oxanilid, 2-Ethoxy-2'-ethyl-oxanilid, N,N'-Bis-(3-dimethylaminopropyl)-oxalamid, 2-Ethoxy-5-tert.butyl-2'-ethyloxanilid und dessen Gemisch mit 2-Ethoxy-2'-ethyl-5,4'-di-tert.butyl-oxanilid, Gemische von ortho- und para-Methoxy- sowie von o- und p-Ethoxy-di-substituierte Oxaniliden.

3. Metalldesaktivatoren, wie z.B. N,N'-Diphenyloxalsäurediamid, N-Salicylal-N'-salicyloylhydrazin, N,N'-Bis-salicyloylhydrazin, N,N'-Bis-(3,5-di-tert.butyl-4-hydroxyphenylpropionyl)-hydrazin, 3-Salicyloylamino-1,2,4-triazol, Bis-benzylidenoxalsäuredihydrazid.

4. Phosphite und Phosphonite, wie z.B. Triphenylphosphit, Diphenylalkylphosphite, Phenyldialkylphosphite, Tri-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Di-(2,4-di-tert.butylphenyl)-pentaerythritdiphosphit, Tristearyl-sorbit-triphosphit, Tetrakis-(2,4-di-tert.butylphenyl)-4,4'-biphenylen-diphosphonit, 3,9-Isodecyloxy-2,4,8,10-tetraxya-3,9-diphosphaspiro-[5,5]-undecan und Tri-(4-hydroxy-3,5-di-tert.butylphenyl)-phosphit.

5. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.

6. Polyamidstabilisatoren, wie z.B. Kupfersalze in Kombination mit Jodiden und/oder Phosphorverbindungen und Salze des zweiwertigen Mangans.

7. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

8. Nucleierungsmittel, wie z.B. 4-tert.Butylbenzoesäure, Adipinsäure, Diphenylessigsäure.

9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Russ, Graphit.

10. Sonstige Zusätze, wie z.B. Weichmacher, Gleitmittel, Emulgatoren, Pigmente, Optische Aufheller, Flammschutzmittel, Antistatika, Treibmittel.

11. Aminoaryl Derivate wie beispielsweise: Phenyl-1-naphthylamin, Phenyl-2-naphthylamin, N,N'-Diphenyl-p-phenylendiamin, N,N'-Di-2-naphthyl-p-phenylendiamin, N,N'-Dinaphthyl-p-phenylendiamin, 6-Ethoxy-2,2,4-trimethyl-1,2-dihydrochinolin, 6-Dodecyl-2,2,4-trimethyl-1,2-dihydrochinolin, Mono- und Dooctyliminodibenzyl, polymerisiertes 2,2,4-Trimethyl-1,2-dihydrochinolin, octyliertes Diphenylamin, nonyliertes Diphenylamin, N-Phenyl-N'-cyclohexyl-p-phenylendiamin, N-Phenyl-N'-isopropyl-p-phenylendiamin, N,N'-Di-sek.octyl-p-phenylendiamin, N-Phenyl-N'-sek.octyl-p-phenylendiamin, N,N'-Di-(1,4-dimethylpentyl)-p-phenylendiamin, N,N'-Di-(1,4-dimethylpentyl)-p-phenylendiamin, N,N'-Dimethyl-N,N'-di-(sek.octyl)-p-phenylendiamin, 2,6-Dimethyl-4-methoxyanilin, 4-Ethoxy-N-sek.butylanilin, Kondensationsprodukt von Diphenylamin und Aceton, Aldol-1-naphthylamin und Phenothiazin.

Die erfindungsgemässen Verbindungen der Formel I können als alleiniger Stabilisator verwendet werden und wirken dann hauptsächlich als Antioxidans oder als Lichtschutzmittel oder sie wirken sowohl als Antioxidans und als Lichtschutzmittel.

Die folgenden Beispiele erläutern die Erfindung ohne sie zu begrenzen. Dabei bedeuten Teile Gewichtsteile soweit nicht anderweitig beschrieben.

Beispiel 1

Herstellung von N,N'-Hexamethylen-bis-[2-(3,5-di-tert.butyl-4-hydroxyphenylthio)-succinimid]

Zu einer Lösung aus 11,05 g N,N'-Hexamethylen-bis-(maleinimid) in 50 ml Tetrahydrofuran wird unter Stickstoff eine Lösung aus 19,07 g 2,6-Di-tert.butyl-4-mercaptophenol und 0,1 g Triethylamin in 100 ml Toluol getropft. Es wird 3 Stunden gerührt, anschliessend das Lösungsmittel abgezogen und der Rückstand aus Cyclohexan umkristallisiert. Man erhält 16,64 g eines weissen Feststoffs vom F.P. 69°C bis 72°C.

Analyse:

| | | | |
|---|---|---|---|
| Ber. | C 67,0 | H 8,0 | N 3,7 |
| Gef. | C 67,4 | H 8,2 | N 3,6 |

Beispiel 2

Herstellung von N-Phenyl-2-(3,5-di-tert.butyl-4-hydroxyphenylthio)-succinimid

Das Verfahren von Beispiel 1 wird wiederholt, indem man 10 g 2,6-Di-tert.butyl-4-mercaptophenol, 7,26 g N-Phenylmaleinimid und 0,73 g Triethylamin verwendet. Das Rohprodukt wird aus Isopropanol umkristallisiert und man erhält 2,3 g (Ausbeute 48%) eines weissen Feststoffs vom F.P. 134°C bis 137°C.

Analyse:

| | | | | |
|---|---|---|---|---|
| Ber. | C 70,0 | H 7,1 | N 3,4 | S 7,8 |
| Gef. | C 69,8 | H 6,8 | N 3,4 | S 7,9 |

Beispiel 3

Herstellung von N-Phenyl-2-(3-tert.butyl-5-methyl-4-hydroxyphenylthio)-succinimid

Das Verfahren von Beispiel 1 wird wiederholt, indem man 9,82 g 2-Tert.butyl-6-methyl-4-mercaptophenol, 8,66 g N-Phenylmaleinimid und 0,5 g Triethylamin einsetzt. Der Rückstand wird aus Heptan/Toluol umkristallisiert und man erhält 16,44 g eines weissen Feststoffs vom F.P. 158°C bis 160°C.

Analyse:

| | | | |
|---|---|---|---|
| Ber. | C 68,3 | H 6,3 | N 3,8 |
| Gef. | C 68,5 | H 6,3 | N 3,7 |

Beispiel 4

Herstellung von N-Methyl-2-(3,5-di-tert.butyl-4-hydroxyphenylthio)-succinimid

Das Verfahren von Beispiel 1 wird wiederholt, indem man 11,92 g 2,6-Di-tert.butyl-4-mercaptophenol, 5,55 g N-Methylmaleinimid und 0,75 g Triethylamin zur Reaktion bringt. Der Rückstand wird aus Toluol/Heptan umkristallisiert und man erhält 21 g eines weissen Feststoffes vom F.P. 274°C bis 278°C.

Analyse:

| | | | |
|---|---|---|---|
| Ber. | C 65,3 | H 7,8 | N 4,0 |
| Gef. | C 65,1 | H 7,6 | N 4,4 |

Beispiel 5

Herstellung von 1,4-Bis-[2-(3,5-di-tert.butyl-4-hydroxyphenylthio)-succinimido]-benzol

Das Verfahren von Beispiel 1 wird wiederholt, indem 17,8 g 2,6-Di-tert.butyl-4-mercaptophenol, 10 g 1,4-Bis-(maleinimido)-benzol und 0,75 g Triethylamin zur Reaktion gebracht werden. Der Rückstand wird aus Aceton umkristallisiert und man erhält 21 g eines weissen Feststoffs vom F.P. 274°C bis 278°C.

Analyse:

| | | | | |
|---|---|---|---|---|
| Ber. | C 67,7 | H 7,0 | N 3,8 | S 8,6 |
| Gef. | C 67,8 | H 7,3 | N 3,7 | S 8,6 |

Beispiel 6

Herstellung von N-n-Butyl-2-(3,5-di-tert.butyl-4-hydroxyphenylthio)-succinimid

Das Verfahren von Beispiel 1 wird wiederholt, indem man 11,92 g 2,6-Di-tert.butyl-4-mercaptophenol, 7,66 g N-n-Butylmaleinimid und 0,5 g Triethylamin einsetzt. Der Rückstand wird säulenchromatographisch gereinigt und man erhält einen weissen Feststoff vom F.P. 80°C bis 85°C.

Analyse:

| | |
|---|---|
| Ber. | S 8,2 |
| Gef. | S 8,2 |

Beispiel 7

Herstellung von 1,2-Bis-[2-(3,5-di-tert.butyl-4-hydroxyphenylthio)-succinimido]-benzol

Das Verfahren von Beispiel 1 wird wiederholt, indem man 11,92 g 2,6-Di-tert.butyl-4-mercaptophenol, 6,71 g N,N'-o-Phenylendimaleinimid und 0,5 g Triethylamin einsetzt. Der Rückstand wird zweimal aus Heptan/Toluol umkristallisiert und man erhält 7,9 g eines weissen Feststoffs vom F.P. 128°C bis 133°C.

Analyse:

| | | | |
|---|---|---|---|
| Ber. | C 67,7 | H 7,0 | N 3,8 |
| Gef. | C 67,5 | H 6,9 | N 3,7 |

Beispiel 8

Herstellung von N-Dodecyl-2-(3,5-di-tert.butyl-4-hydroxyphenylthio)-succinimid

In Analogie zum Verfahren des Beispiels 1 setzt man 13,27 g N-Dodecylmaleinimid, 11,92 g 2,6-Di-tert.butyl-4-mercaptophenol und 0,5 g Triethylamin um. Das Reaktionsprodukt wird säulenchromatographisch gereinigt und man erhält 10,0 g eines klaren syrupartigen Produkts.

Analyse:

| | | | |
|---|---|---|---|
| Ber. | C 71,5 | H 9,8 | N 2,8 |
| Gef. | C 71,3 | H 9,8 | N 2,9 |

Beispiel 9

Herstellung von 4,4'-Bis-[2-(3,5-di-tert.butyl-4-hydroxyphenylthio)-succinimido]-diphenylmethan

Das Verfahren von Beispiel 1 wird wiederholt, indem man n 9,17 g 4,4'-Bis-(maleinimido)-diphenylmethan, 12,20 g 2,6-Di-tert.butyl-4-mercaptophenol und 0,5 g Triethylamin einsetzt.

Das Reaktionsprodukt wird säulenchromatographisch gereinigt und man erhält 1,3 g eines weissen Feststoffs vom F.P. 103°C bis 110°C.

Analyse:

| | | | |
|---|---|---|---|
| Ber. | C 70,5 | H 7,0 | N 3,4 |
| Gef. | C 70,8 | H 6,9 | N 3,1 |

Beispiel 10

Herstellung von N-Cyclohexyl-2-(3,5-di-tert.butyl-4-hydroxyphenylthio)-succinimid

Das Verfahren von Beispiel 1 wird wiederholt, indem man 11,95 g 2,6-Di-tert.butyl-4-mercaptophenol, 8,6 g N-Cyclohexylmaleinimid und 0,5 g Triethylamin zur Reaktion bringt. Der Rückstand wird aus Heptan/Toluol umkristallisiert und man erhält 10,5 g eines weissen Feststoffs vom F.P. 94°C bis 98°C.

Analyse:

| | | | | |
|---|---|---|---|---|
| Ber. | C 69,0 | H 8,5 | N 3,4 | S 7,7 |
| Gef. | C 69,0 | H 8,3 | N 3,4 | S 7,7 |

Beispiel 11

In diesem Beispiel wird die stabilisierende Wirkung der erfindungsgemässen Verbindungen in schlagfestem Polystyrol demonstriert.

a) Herstellung der Proben: Man stellt eine Lösung von 8 Gew.% Polybutadien-Kautschuk (Firestone DIENE 55) in monomerem Styrol her, indem man beide Komponenten in einer Quetschmühle mischt. Gleichzeitig wird in diesem Arbeitsgang der jeweils gewünschte Anteil des zu testenden Stabilisators zu der Lösung gegeben. Schliesslich fügt man noch 500 ppm Zn-Stearat hinzu, um später die polymerisierte Probe leicht aus dem Reaktionsgefäss ablösen zu können. Die Polymerisation wird in einem Gefäss durchgeführt, dass mit einem Wendelrührer ausgerüstet ist. Da die meisten IPS Blockpolymerisationen

thermisch initiiert werden, wird in der vorliegenden Laboratoriumsversion kein Starter zugesetzt. Die Reaktion wird unter Stickstoff und unter Erwärmen durchgeführt. Der Reaktor wird innerhalb einer halben Stunde auf 121°C erwärmt, und diese Temperatur wird aufrecht erhalten, bis zwischen 30 und 35% des Monomeren umgesetzt sind (Zeitdauer ca. 2,5 Stunden). Während der Polymerisation wird heftig gerührt, und die Rührgeschwindigkeit wird so eingestellt, dass Polymerpartikel von 2 bis 4 µm Grösse entstehen. Anschliessend werden die Reaktionsgefässe dem Reaktor entnommen, im Stickstoffstrom geöffnet und in ein Wirbelschichtsandbad gestellt, um die Polymerisation zu vervollständigen. Die Gefässe werden im Sandbad folgendermassen erhitzt: eine Stunde bei 100°C um die Anfangstemperatur vorzugeben, eine weitere Stunde, um 140°C zu erreichen und anschliessend weitere 8 Stunden, wobei die Temperatur jede Stunde um 10°C erhöht wird, bis schliesslich ein Maximum von 220°C erreicht ist. Nach dem Abkühlen werden die Polymerisationsgefässe zerbrochen und das Glas entfernt. Ein einziger Polymerblock wiegt im Mittel etwas über 600 g. Der Block wird in einem Vakuumofen bei 200°C für 45 Minuten bei einem Druck von 1,33 mbar belassen, um flüchtige Bestandteile zu entfernen. Nach dieser Behandlung wird der Block sofort in einer heizbaren hydraulischen Presse bei 205°C zwischen zwei Aluminiumfolien zu einem dicken Stab gepresst (drei Minuten Erhitzen, fünf Minuten ungeheizt).

Der Stab wird mittels einer Handsäge zerkleinert und anschliessend granuliert. Alle Polymermischungen werden bei 205°C extrudiert und dann zerkleinert. Die Pellets werden unter Druck bei 205°C in dehnbare 3,175 mm dicke Streifen verformt. Diese Probestreifen werden dann bei 150°C in einem Umluftofen gealtert, wobei sie auf Glasplatten, die sich wiederum auf rotierenden Trägern befinden, aufgebracht sind. Eine weitere Charge von Probestreifen wird auf dieselbe Weise bei 80°C im Ofen gealtert. Nach bestimmten Zeitintervallen wird der Yellowness Index der Proben gemäss ASTM D-1925-63T bestimmt, und es wird die Dehnbarkeit bestimmt (Instron Tensile Testing Apparatus, Instron Engineering Corporation, Massachusetts; Ziehgeschwindigkeit: 5 mm/Minute).

In der folgenden Tabelle 1a sind die Ergebnisse der Dehnbarkeitsmessungen und der Messungen des Yellowness Index von bei 80°C ofengealterten Proben angegeben, während Tabelle 1b Messwerte von bei 150°C ofengealterten Proben enthält.

Tabelle 1a
Dehnbarkeitsmessungen und Yellowness Index von bei 80°C ofengealterten Proben mit und ohne Stabilisator

| Additiv | Stabilisatormenge (Gew.%) | Dehnbarkeit der Proben (%) und Dauer der Hitzeeinwirkung (h) | | | | |
|---|---|---|---|---|---|---|
| | | 0 h | 300 h | 600 h | 900 h | 1200 h |
| Produkt von Beispiel 2 | 0,1 | 40 | 28 | 18 | 8 | 4 |
| – | – | 33 | 9 | 3 | 3 | 3 |

| Additiv | Stabilisatormenge (Gew.%) | Yellowness Index und Dauer der Hitzebelastung (h) | | | | |
|---|---|---|---|---|---|---|
| | | 0 h | 300 h | 600 h | 900 h | 1200 h |
| Produkt von Beispiel 2 | 0,1 | –3 | 1 | 11 | 28 | 44 |
| – | – | 7 | 14 | 45 | 59 | – |

Tabelle 1b
Dehnbarkeitsmessungen und Yellowness Index von bei 150°C ofengealterten Proben mit und ohne Stabilisator

| Additiv | Stabilisatormenge (Gew.%) | Dehnbarkeit der Probe (%) und Dauer der Hitzebelastung (h) | | | | |
|---|---|---|---|---|---|---|
| | | 0 h | 0,5 h | 1 h | 1,5 h | 2 h |
| Produkt von Beispiel 2 | 0,1 | 40 | 42 | 14 | 13 | 10 |
| – | – | 33 | 7 | 7 | 3 | 3 |

| Additiv | Stabilisator-menge (Gew.%) | Yellowness Index und Dauer der Hitzebelastung (h) | | | | |
|---|---|---|---|---|---|---|
| | | 0 h | 0,5 h | 1 h | 1,5 h | 2 h |
| Produkt von Beispiel 2 | 0,1 | -3 | -3 | 2 | 4 | 10 |
| – | – | 7 | 18 | 30 | 38 | 43 |

Beispiel 12

Unstabilisiertes Polypropylenpulver (Hercules Profax 6501) wird mit einer bestimmten Menge eines Additivs gemischt. Diese Mischungen werden anschliessend bei 182°C 5 Minuten lang auf einem Mischwalzwerk plastifiziert. Anschliessend wird die stabilisierte Polypropylenfolie vom Walzwerk abgelöst und abkühlen gelassen. Die Folie wird in Stücke geschnitten und auf einer hydraulischen Presse bei 220°C und 12 bar zu einer 0,127 mm dicken Probe verformt. Diese Probe wird ultraviolettem Licht bis zur Zersetzung ausgesetzt. Als Zersetzungszeitpunkt wird diejenige Anzahl von Stunden gewertet, bei der die IRspektroskopisch gemessene dekadische Extinktion der Carbonylbande 50% des Anfangswertes erreicht. Die Messdaten sind in der folgenden Tabelle 2 dargestellt:

Tabelle 2

| Additiv (Produkt von Beispiel) | Stabilisatormenge (Gew.%) | Zahl der Stunden bis zur Zersetzung der bestrahlten Proben |
|---|---|---|
| 1 | 0,2 | 500 |
| 2 | 0,2 | 400 |
| 6 | 0,2 | 400 |
| 8 | 0,2 | 380 |
| 9 | 0,2 | 400 |
| – | – | 200–300 |

Beispiel 13

Es wird die Oxidationsstabilität von Polypropylenproben gemäss Beispiel 12 gemessen, die 0,2 Gew.% der Verbindung von Beispiel 1 als Stabilisator enthalten. Ferner wird die Oxidationsstabilität von Polypropylenproben gemäss Beispiel 12 gemessen, die eine synergistisch wirkende Formulierung bestehend aus 0,1 Gew.% des Stabilisators von Beispiel 1 und aus 0,3 Gew.% Distearylthiodipropionat enthalten. Als Proben werden 0,635 mm dicke Teststreifen verwendet, die im Umluftofen bei 150°C an der Luft gealtert werden. Die Teststreifen werden dann als zersetzt betrachtet, wenn erste Zerfallserscheinungen (Brüche, braune Ecken) sichtbar werden. Zu Beginn und während des Versuchs wird die Farbe der Proben nach der Standard Methode von Gardner (ASTM D 1544-68) bestimmt. Die Ergebnisse sind in der nachfolgenden Tabelle 3 aufgelistet:

Tabelle 3

| Additiv | Stabilisatormenge (Gew.%) | Zersetzungszeitpunkt (Stunden) | Farbmessung nach Gardner | |
|---|---|---|---|---|
| | | | Farbwert | Testdauer (h) |
| DSTDP*) | 0,3 | <20 | 0 | 0 |
| | | | 2 | bei Zersetzung |
| Produkt von Beispiel 1 | 0,2 | 650 | 0 | 0 |
| | | | 1 | 100 |
| | | | 1 | bei Zersetzung |
| Produkt von Beispiel 1 | 0,1 | 1120 | 0 | 0 |
| | | | 1 | 100 |
| + DSTDP*) | 0,3 | | 1 | bei Zersetzung |

*) Distearyl-thiodipropionat

**Patentansprüche**

1. Verbindungen der Formel I

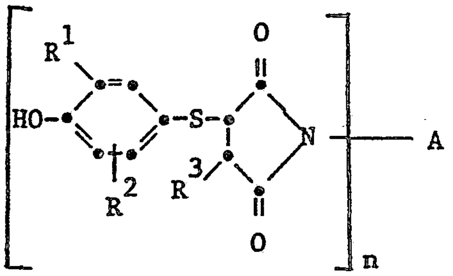

(I),

worin $R^1$ und $R^2$ unabhängig voneinander Wasserstoff, $C_1$–$C_{18}$Alkyl, $C_5$–$C_6$Cycloalkyl, Phenyl oder mit $C_1$–$C_{12}$Alkyl substituiertes Phenyl bedeuten, worin $R^3$ Wasserstoff oder $C_1$–$C_3$Alkyl bedeutet, n 1, 2 oder 3 ist, und worin A, je nach Wert von n, folgende Bedeutung zukommt:

a) wenn n 1 ist, bedeutet A Phenyl, mit ein oder zwei $C_1$–$C_{18}$Alkylgruppen substituiertes Phenyl, $C_1$–$C_{30}$Alkyl, $C_5$ oder $C_6$ Cycloalkyl, oder A ist ein Rest vom Typ E–(G–T)$_y$–, worin E $C_1$–$C_{18}$Alkyl bedeutet, G -O- oder -NH- ist, T Ethylen, Propylen oder 1,4-Butylen bedeutet und Y eine Zahl von 1 bis 30 ist;

b) wenn n 2 ist, bedeutet A Phenylen, durch ein oder zwei $C_1$–$C_{12}$-Alkylgruppen substituiertes Phenylen, $C_1$–$C_{12}$Alkylen, $C_5$ oder $C_6$ Cycloalkylen, Phenylen-L-Phenylen, worin L $C_1$–$C_4$Alkylen bedeutet, oder A ist -T-(G-T)$_z$-, worin T Ethylen, Propylen oder 1,4-Butylen bedeutet, G -O- oder -NH- ist, und z eine Zahl von 1 bis 30 ist;

c) wenn n 3 ist, bedeutet A Benzol-1,2,3-, -1,2,4- oder -1,3,5-triyl oder N(–$R^4$–)$_3$, worin $R^4$ $C_2$–$C_4$Alkylen bedeutet.

2. Verbindungen der Formel I gemäss Anspruch 1, worin $R^1$ $C_4$–$C_8$Alkyl bedeutet.

3. Verbindungen der Formel I gemäss Anspruch 2, worin $R^1$ tert.Butyl, tert.Pentyl oder 1,1,3,3-Tetramethylbutyl bedeutet.

4. Verbindungen der Formel I gemäss Anspruch 1, worin sich die Gruppe $R^2$ in o-Position zur Hydroxygruppe des Phenylrings befindet.

5. Verbindungen der Formel I gemäss Anspruch 1, worin $R^2$ $C_4$–$C_8$tert.Alkyl bedeutet.

6. Verbindungen der Formel I gemäss Anspruch 1, worin n 1 ist und A Phenyl oder $C_1$–$C_{12}$Alkyl bedeutet.

7. Verbindungen der Formel I gemäss Anspruch 1, worin n 2 ist und A Hexamethylen, Phenylen oder 4,4-Methylendiphenylen bedeutet.

8. Die Verbindung N,N-Hexamethylen-bis-[2-(3,5-di-tert.butyl-4-hydroxyphenylthio)-succinimid] gemäss Anspruch 1.

9. Die Verbindung N-Phenyl-2-(3,5-di-tert.butyl-4-hydroxyphenylthio)-succinimid gemäss Anspruch 1.

10. Die Verbindung N-n-Butyl-2-(3,5-di-tert.butyl-4-hydroxyphenylthio)-succinimid gemäss Anspruch 1.

11. Die Verbindung N-Dodecyl-2-(3,5-di-tert.butyl-4-hydroxyphenylthio)-succinimid gemäss Anspruch 1.

12. Die Verbindung 4,4-Bis-[2-(3,5-di-tert.butyl-4-hydroxyphenylthio)-succinimido]-diphenylmethan gemäss Anspruch 1.

13. Zusammensetzungen enthaltend ein gegen oxidativen, thermischen oder strahlungsinduzierten Abbau empfindliches organisches Material und mindestens eine Verbindung der Formel I gemäss Anspruch 1.

14. Zusammensetzung gemäss Anspruch 13, worin das organische Material ein Polymeres ist.

15. Zusammensetzung gemäss Anspruch 14, worin das Polymere ein polyolefinisches Homo- oder Copolymer bedeutet.

16. Zusammensetzungen gemäss Anspruch 14, worin das Polymere ein Homopolymer, Copolymer oder Terpolymer von Styrol ist.

17. Ein Verfahren zum Stabilisieren von organischem Material gegen oxidativen, thermischen oder strahlungsinduzierten Abbau, dadurch gekennzeichnet, dass man dem Material mindestens eine Verbindung der Formel I gemäss Anspruch 1 zusetzt.

**Claims**

1. A compound of the formula

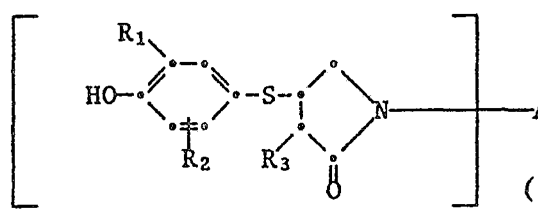

(I),

wherein $R_1$ and $R_2$ are each independently of the other hydrogen, $C_1$–$C_{18}$alkyl, $C_5$–$C_6$cycloalkyl, phenyl or phenyl substituted by $C_1$–$C_{12}$alkyl; $R_3$ is hydrogen or $C_1$–$C_3$alkyl; n is 1, 2 or 3; and A, depending on the value of n, has the following meanings:

a) when n is 1, A is phenyl, phenyl substituted by one or two $C_1$–$C_{18}$alkyl groups, or is $C_1$–$C_{30}$alkyl, $C_5$–$C_6$cycloalkyl; or is E–(G–T)$_y$– where E is $C_1$–$C_{18}$alkyl, G is -O- or -NH-, T is ethylene, propylene or 1,4-butylene and Y is 1 to 30;

b) when n is 2, A is phenylene, phenylene substituted by one or two $C_1$–$C_{12}$alkyl groups or is $C_1$–$C_{12}$alkylene, $C_5$–$C_6$cycloalkylene, phenylene-L-phenylene, where L is $C_1$–$C_4$alkylene, or is -T- (G-T)$_z$- where T is ethylene, propylene or 1,4-butylene, G is -O- or -NH-, and z is 1 to 30;

c) when n is 3, A is 1,2,3-benzenetriyl, 1,2,4-benzetneriyl, 1,3,5,-benzenetriyl or N(–$R_4$–)$_3$, where $R_4$ is $C_2$–$C_4$alkylene.

2. A compound of formula I according to claim 1, wherein $R_1$ is $C_4$–$C_8$alkyl.

3. A compound of formula I according to claim 2, wherein $R_1$ is tert-butyl, tert-pentyl or 1,1,3,3-tetramethylbutyl.

4. A compound of formula I according to claim 1, wherein $R_2$ is in the ortho position to the hydroxyl group in the phenyl ring.

5. A compound of formula I according to claim 1, wherein $R_2$ is $C_4$–$C_8$tert-alkyl.

6. A compound of formula I according to claim 1, wherein n is 1 and A is phenyl or $C_1$–$C_{12}$alkyl.

7. A compound of formula I according to claim 1, wherein n is 2 and A is hexamethylene, phenylene or 4,4′-methylenediphenylene.

8. N,N′-Hexamethylene bis[2-(3,5-di-tert-butyl-4-hydroxyphenylthio)succinimide] according to claim 1.

9. N-Phenyl-2-(3,5-di-tert-butyl-4-hydroxyphenylthio)succinimide according to claim 1.

10. N-n-Butyl-2-(3,5-di-tert-butyl-4-hydroxyphenylthio)succinimide according to claim 1.

11. N-Dodecyl-2-(3,5-di-tert-butyl-4-hydroxyphenylthio)succinimide according to claim 1.

12. 4,4′-Bis[2-(3,5-di-tert-butyl-4-hydroxyphenylthio)succinimido]-diphenylmethane according to claim 1.

13. A composition of matter comprising an organic material subject to oxidative, thermal and actinic degradation and at least one compound of formula I according to claim 1.

14. A composition according to claim 13, wherein the organic material is a polymer.

15. A composition according to claim 14, wherein the polymer is a polyolefin homopolymer or copolymer.

16. A composition according to claim 14, wherein the polymer is a styrene homopolymer, copolymer or terpolymer.

17. A method of stabilizing an organic material against oxidative, thermal and actinic degradation, which comprises incorporating into said organic material at least one compound of formula I according to claim 1.

**Revendications**

1. Composés répondant à la formule I:

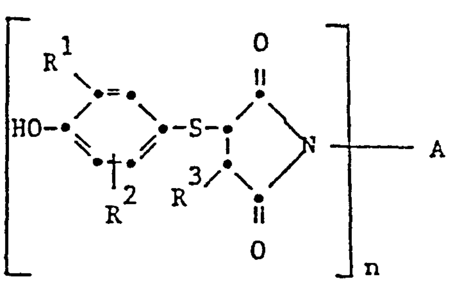

(I)

dans laquelle:

$R^1$ et $R^2$ représentent chacun, indépendamment l'un de l'autre, l'hydrogène, un alkyle en $C_1$–$C_{18}$, un cycloalkyle en $C_5$ ou $C_6$, un phényle ou un phényle porteur d'un alkyle en $C_1$–$C_{12}$,

$R^3$ représente l'hydrogène ou un alkyle en $C_1$–$C_3$, n est égal à 1, à 2 ou à 3 et A représente:

a) lorsque n est égal à 1 un phényle, un phényle porteur d'un ou de deux alkyles en $C_1$–$C_{18}$, un alkyle en $C_1$–$C_{30}$, un cycloalkyle en $C_5$ ou $C_6$ ou un radical E–(G–T)$_y$– dans lequel E représente un alkyle en $C_1$–$C_{18}$, G représente –O– ou –NH–, T représente un radical éthylène, propylène ou butylène-1,4 et Y représente un nombre de 1 à 30,

b) lorsque n est égal à 2 un phénylène, un phénylène porteur d'un ou de deux alkyles en $C_1$–$C_{12}$, un alkylène en $C_1$–$C_{12}$, un cycloalkylène en $C_5$ ou $C_6$, un radical phénylène-L-phénylène dans lequel L représente un alkylène en $C_1$–$C_4$, ou A représente un radical –T–(G–T)$_z$– dans lequel T représente un radical éthylène, propylène ou butylène-1,4, G représente –O– ou –NH– et z désigne un nombre de 1 à 30, et

c) lorsque n est égal à 3 un radical benzènetriyle-1,2,3, -1,2,4 ou -1,3,5 ou un radical $N(-R^4-)_3$ dans lequel $R^4$ représente un alkylène en $C_2$–$C_4$.

2. Composés de formule I selon la revendication 1 dans lesquels $R^1$ représente un radical alkyle contenant de 4 à 8 atomes de carbone.

3. Composés de formule I selon la revendication 2 dans lesquels $R^1$ représente un radical tert-butyle, tert-pentyle ou tétraméthyl-1,1,3,3 butyle.

4. Composés de formule I selon la revendication 1 dans lesquels le radical $R^2$ se trouve en position ortho relativement au radical hydroxy du noyau phényle.

5. Composés de formule I selon la revendication 1 dans lesquels $R^2$ représente un radical alkyle tertiaire contenant de 4 à 8 atomes de carbone.

6. Composés de formule I selon la revendication 1 dans lesquels n est égal à 1 et A représente un radical phényle ou un radical alkyle contenant de 1 à 12 atomes de carbone.

7. Composés de formule I selon la revendication 1 dans lesquels n est égal à 2 et A représente un radical hexaméthylène, phénylène ou méthylène-4,4′ diphénylène.

8. Composé selon la revendication 1, en l'espèce le N,N′-hexaméthylène-bis-[(di-tert-butyl-3,5 hydroxy-4 phénylthio)-2 succinimide].

9. Composé selon la revendication 1, en l'espèce le N-phényl (di-tert-butyl-3,5 hydroxy-4 phénylthio)-2 succinimide.

10. Composé selon la revendication 1, en l'espèce le N-n-butyl (di-tert-butyl-3,5 hydroxy-4 phénylthio)-2 succinimide.

11. Composé selon la revendication 1, en l'espèce le N-dodécyl (di-tert-butyl-3,5 hydroxy-4 phénylthio)-2 succinimide.

12. Composé selon la revendication 1, en l'espèce le bis-[(di-tert-butyl-3,5 hydroxy-4 phénylthio)-2 succinimido]-4,4′ diphénylméthane.

13. Compositions qui contiennent une matière organique risquant d'être dégradée sous l'action de l'oxydation, de la chaleur ou d'un rayonnement, et au moins un composé de formule I selon la revendication 1.

14. Composition selon la revendication 13 dans laquelle la matière organique est un polymère.

15. Composition selon la revendication 14 dans laquelle le polymère est un homopolymère ou un copolymère polyoléfinique.

16. Compositions selon la revendication 14 dans lesquelles le polymère est un homopolymère, un copolymère ou un terpolymère du styrène.

17. Procédé pour stabiliser des matières organiques contre la dégradation due à l'oxydation, à la chaleur ou à un rayonnement, procédé caractérisé en ce qu'on ajoute à la matière au moins un composé de formule I selon la revendication 1.